# EUROPEAN PATENT APPLICATION

(11) **EP 2 716 213 A1**
(43) Date of publication of application: **09.04.2014**
(21) Application number: 12187021.6
(22) Date of filing: 02.10.2012
(51) Int. Cl.: A61B 5/145, A61B 5/00

(54) **Improved measuring in a mobile terminal**

(71) Applicant: Doro AB, 226 43 Lund (SE)
(72) Inventor: Krokstäde, Calle, Tai Wai, Sha Tin (CN); Persson, Madelene, 271 57 Ystad (SE)
(74) Representative: Ström & Gulliksson AB

(57) **Abstract**

A mobile terminal (100, 200, 500) comprising a memory (240), a measuring device (540) and a plurality of components (120, 220, 520, 530, 230, 210), one component being a controller (210). The mobile terminal is configured to operate in a terminal functional mode and determine that a measuring mode is to be activated and enter said measuring mode. While in the measuring mode the mobile terminal reduces the activity of at least one of said components (120, 220, 520, 530, 230, 210) to reduce the interference caused by the components (120, 220, 520, 530, 230, 210) to the measuring device (540). The mobile terminal causes said measuring device (540) to perform a measurement and resumes the activity of said at least one component (120, 220, 520, 530, 230, 210) as the measurement has been made.

## Description

### TECHNICAL FIELD

This application relates to a method, a mobile terminal and a computer-readable storage medium for improved measuring in a mobile terminal, and in particular to a method, a mobile communications terminal and a computer-readable storage medium for improved measuring of a blood glucose level in a mobile communications terminal incorporating a blood glucose measuring device.

### BACKGROUND

In today's society where mobile communications terminals are becoming more and more commonplace, attempts have been made to combine the functionality of the mobile communications terminal with a health functionality, one example being a blood glucose meter. However, there exists a problem when measuring the blood glucose level in that the blood glucose measuring is sensitive to external disturbances, such as radiation from electronic circuitry, and the blood glucose measuring is also sensitive to the temperature and temperature changes.

One system which incorporates a blood glucose measuring device in a mobile communications terminal is disclosed in the European patent application published as EP 1 859 533 that discloses a mobile telephone provided with an integrated measuring module, which is shielded against electromagnetic waves emitted by said mobile telephone. A test strip used in the measuring module and shielded against electromagnetic waves is also disclosed. In this system the blood glucose measuring device is shielded by a specifically adapted shield to prevent Radio Frequency radiation from interfering with the measuring. Such shields are not able to block out all the radiation while still being lightweight and easy to install in a portable device which has strict requirements on a low weight and a small size.

Another system is disclosed in the international patent application published as WO 03/094713 that discloses a blood sugar test device and a method for transmitting a measured blood sugar level to a blood sugar level administration server on the Internet. In one embodiment, blood sugar test function is integrated in a mobile terminal. In another embodiment, a blood sugar test adaptor is connected to a connection terminal of a mobile terminal, the adaptor being equipped with minimal functions to perform blood sugar tests. In addition, both the mobile terminal with integrated blood sugar test function and the mobile terminal connected to the above blood sugar test adaptor can transmit measured blood sugar level to the blood sugar level administration server. A strip case is provided for at an outer surface of the mobile terminal itself in the former, while a strip case is located at an outer surface of the adaptor in the latter. This system is adapted to measure the surrounding temperature and take that into account when determining the result of the measurement. This requires that specific hardware is installed in the mobile communications terminal which is costly, consumes valuable space and also draws more power.

A third system is disclosed in the American patent application published as US 2009/069744 that discloses a glucose meter module integrated into a holster device that can securely accommodate another device such as a portable server device or an insulin pump is described. The glucose measuring module and the health device communicate with each other by a short range wireless modality. In the case in which the accommodated device is a server, such as personal digital assistant or cell phone, the device stores data in a memory, displays data on a visual display, and can wirelessly transmit such data to other devices within a personal area network. In the case where the accommodated device is a cell phone, the phone can further transmit data to remote sites. In the case where the accommodated device is an insulin pump, wirelessly received data are stored in a memory, are available for visual display on the insulin pump, and can be incorporated into the electronic processes that regulate the performance of the pump. Removing the blood glucose measuring device from the other components requires that the resulting device is quite large to accommodate the distance between the blood glucose measuring device and the components. Such an arrangement is thus not suited for small portable devices in which the available space is sparse.

There is thus a need for an improved manner of performing measurements in a mobile communications terminal and also other mobile terminals that does not require specifically adapted hardware or consumes available terminal space.

### SUMMARY

It is an object of the teachings of this application to overcome the problems listed above by providing a mobile terminal comprising a memory, a measuring device and a plurality of components, one component being a controller, wherein said controller is configured to operate in a terminal functional mode, determine that a measuring mode is to be activated and enter said measuring mode, reduce the activity of at least one component, cause said measuring device to perform a measurement, and resume the activity of said at least one of said components and thereby reducing the interference caused by the components to the measuring device during the measurement.

In one embodiment the mobile terminal is a mobile communications terminal. In one embodiment the measuring device is a blood glucose measuring device.

This enables a mobile communications terminal to incorporate a blood glucose measuring device and provide reliable and accurate measurements.

It is a further object of the teachings of this application to overcome the problems listed above by providing a method for use in a mobile terminal comprising a memory, a measuring device and a plurality of components, said method comprising operating in a terminal functional mode, determining that a measuring mode is to be activated and enter said measuring mode, reducing the activity of at least one of said components, causing said measuring device to perform a measurement, and resuming the activity of said at least one component.

It is a further object of the teachings of this application to overcome the problems listed above by providing a computer readable medium comprising instructions that when loaded into and executed by a controller, such as a processor, cause the execution of a method according to herein.

Other features and advantages of the disclosed embodiments will appear from the attached detailed disclosure.

### BRIEF DESCRIPTION OF DRAWINGS

The invention will be described in further detail under reference to the accompanying drawings in which:
Figure 1 shows a schematic view of a mobile terminal according to one embodiment of the teachings of this application;
Figure 2 shows a schematic view of the general structure of a mobile terminal according to one embodiment of the teachings of this application;
Figure 3 shows a schematic view of a telecommunications network comprising a mobile communications terminal according to one embodiment of the teachings of this application;
Figure 4 shows a schematic view of a computer-readable medium according to one embodiment of the teachings of this application;
Figures 5A and 5B each show a schematic view of a mobile terminal according to an embodiment of the teachings of this application; and
Figure 6 shows a flow chart for a general method according to an embodiment of the teachings of this application.

### DETAILED DESCRIPTION

The disclosed embodiments will now be described more fully hereinafter with reference to the accompanying drawings, in which certain embodiments of the invention are shown. This invention may, however, be embodied in many different forms and should not be construed as limited to the embodiments set forth herein; rather, these embodiments are provided by way of example so that this disclosure will be thorough and complete, and will fully convey the scope of the invention to those skilled in the art. Like numbers refer to like elements throughout.

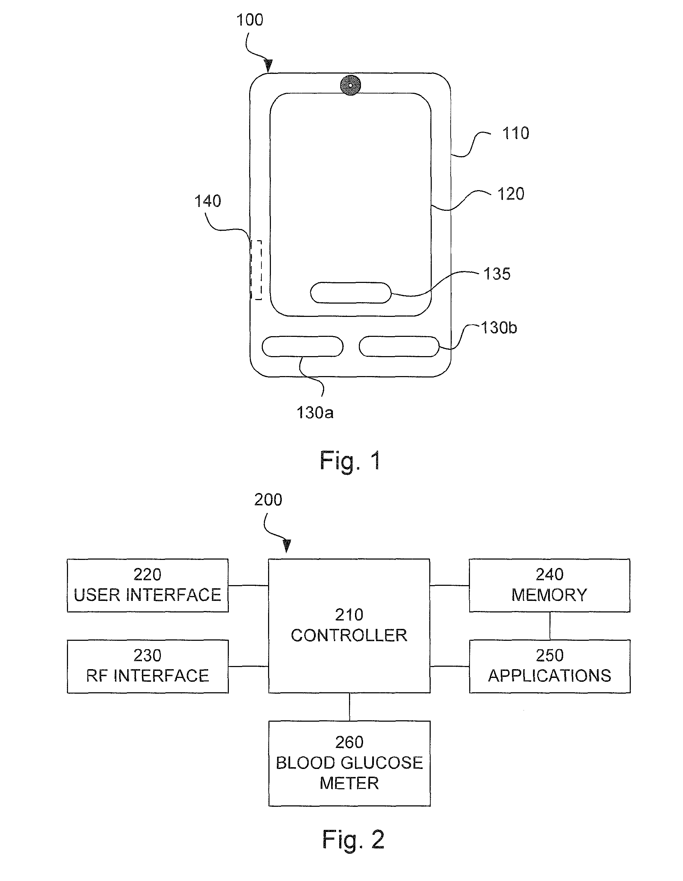

Figure 1 shows a schematic overview of a mobile terminal 100 adapted according to the teachings herein. In the embodiment shown the mobile terminal is a mobile communications terminal and in this specific example it is a mobile phone 100. In other embodiments the mobile communications terminal 100 is a personal digital assistant, a media player, a location finding device or any hand-held device capable of communicating with other devices. It should be noted that in other embodiments the mobile terminal can be a general health monitoring device incorporating a measuring device.

The mobile phone 100 comprises a housing 110 in which a display 120 is arranged. In one embodiment the display 120 is a non-touch display. In other embodiments the display 120 is a touch display. Furthermore, the mobile phone 100 comprises two hard-wired keys 130. In this embodiment there are two softkeys 130a and 130b, but any number of keys, including none, is possible and depends on the design of the mobile phone 100. In one embodiment the mobile phone 100 is configured to display and operate a virtual key 135 on the touch display 120. It should be noted that the number of virtual keys 135 are dependent on the design of the mobile phone 100 and an application that is executed on the mobile phone 100. In one embodiment the communications terminal 100 comprises an ITU-T keypad or a QWERTY (or equivalent) keypad in addition to or as an alternative to a touch-sensitive display. In an embodiment where the keypad is an alternative to a touch-sensitive display, the display 120 is a non-touch-sensitive display.

The mobile phone 100 further comprises a blood glucose measuring device 140. In figure 1 the blood glucose measuring device 140 is illustrated with a dashed box indicating the location of an opening of the blood glucose measuring device 140 arranged to receive a test strip carrying a substance to be tested by the blood glucose measuring device 140.

Figure 2 shows a schematic view of the general structure of a mobile terminal which may be a mobile communications terminal such as a mobile phone 100 according to figure 1. The mobile terminal 200 comprises a controller 210 which is responsible for the overall operation of the mobile terminal and is preferably implemented by any commercially available CPU ("Central Processing Unit"), DSP ("digital signal processor") or any other electronic programmable logic device, or a combination of such processors and/or other electronic programmable logic device. The controller 210 may be implemented using instructions that enable hardware functionality, for example, by using executable computer program instructions in a general-purpose or special-purpose processor that may be stored on a computer readable storage medium (disk, memory etc) 240 to be executed by such a processor. The controller 210 is configured to read instructions from the memory 240 and execute these instructions to control the operation of the mobile terminal 200. The memory 240 may be implemented using any commonly known technology for computer-readable memories such as ROM, RAM, SRAM, DRAM, CMOS, FLASH, DDR, EEPROM memory, flash memory, hard drive, optical storage or any combination thereof. The memory 240 is used for various purposes by the controller 210, one of them being for storing application data and various software modules in the mobile terminal.

The software modules include a real-time operating system, drivers for a user-machine interface 220, an application handler as well as various applications 250. The applications 250 are sets of instructions that when executed by the controller 210 control the operation of the mobile terminal 200. The applications 250 can include a messaging application for short messaging service (SMS), multimedia messaging service (MMS) and electronic mail, a media player application, as well as various other applications 250, such as applications for voice calling, video calling, web browsing, document reading and/or document editing, an instant messaging application, a phonebook application, a calendar application, a control panel application, one or more video games, a notepad application, location finding applications, etc.

The mobile terminal 200 further comprises a measuring device 260, for instance the blood glucose measuring device 140 shown in figure 1. In this embodiment the measuring device 260 is a blood glucose measuring device 260. The blood glucose measuring device 260 is arranged to receive a test strip which is arranged to carry a test substance. In an actual measuring situation the test substance is the blood of a test subject. In a calibration or test procedure the test substance is a (special) testing substance having known qualities. The test strip is inserted into the blood glucose measuring device 260 and the blood glucose measuring device measures the blood glucose level in the substance. Blood glucose measuring devices are commonly known and the manner in which a blood glucose measuring device operates should be well-known to a reader skilled in blood glucose measuring devices.

The mobile terminal 200 further comprises a user interface 220, which in the mobile phone 100 of figure 1 is comprised of the display 120, the keys 130, 135, a microphone and a loudspeaker. The user interface (UI) 220 also includes one or more hardware controllers, which together with the user interface drivers cooperate with the display 120, keypad 130, as well as various other I/O devices such as vibrator, ringtone generator, LED indicator, etc. As is commonly known, the user may operate the mobile terminal through the man-machine interface thus formed.

The mobile terminal 200 may further comprise a radio frequency interface 230, which is adapted to allow the mobile communications terminal to communicate with other communications terminals in a radio frequency band through the use of different radio frequency technologies. Examples of such technologies are W-CDMA, GSM, UTRAN, LTE and NMT to name a few. The controller 210 is configured to operably execute the applications 250, such as the voice call and message handling applications, through the RF interface 230 and software stored in the memory 240. The software includes various modules, protocol stacks, drivers, etc. to provide communication services (such as transport, network and connectivity) for the RF interface 230, and optionally a Bluetooth interface, an IEEE 802.11 standard interface (wifi) and/or an IrDA interface for short range, local connectivity. The RF interface 230 comprises an internal or external antenna as well as appropriate radio circuitry for establishing and maintaining a wireless link to a base station. As is well known to a person skilled in the art, the radio circuitry comprises a series of analogue and digital electronic components, together forming a radio receiver and transmitter. These components include, for example, band pass filters, amplifiers, mixers, local oscillators, low pass filters, AD/DA converters, etc.

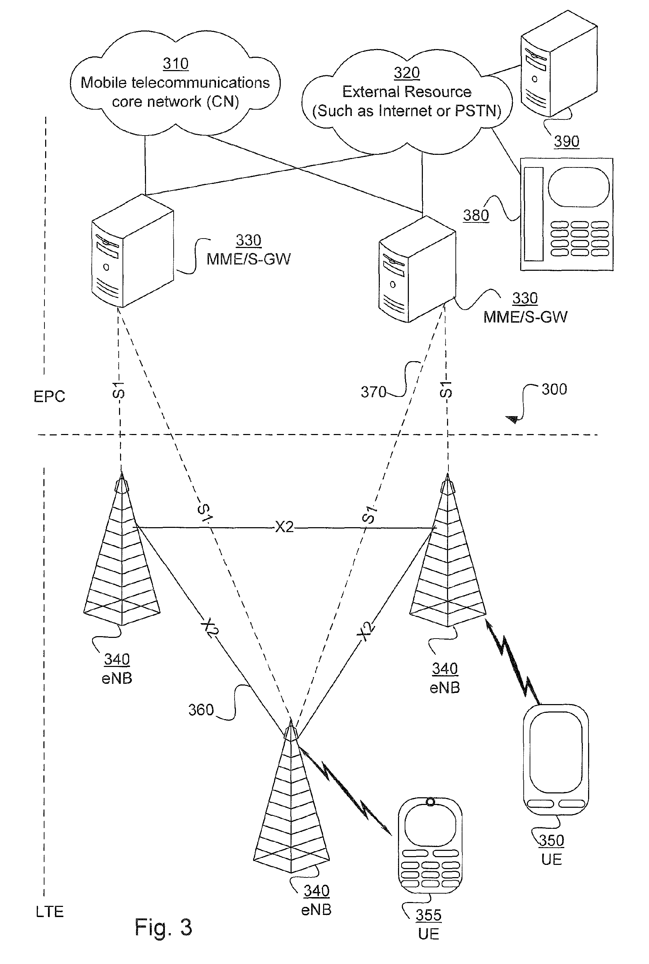

Figure 3 shows a schematic view of the general structure of a telecommunications system 300 according to the teachings herein. In the telecommunication system of figure 3, various telecommunications services such as cellular voice calls, www/wap browsing, cellular video calls, data calls, facsimile transmissions, music transmissions, still image transmissions, video transmissions, electronic message transmissions and electronic commerce may be performed between a mobile terminal 100, 200, 350, 355 (which in this embodiment is a mobile communications terminal) according to the disclosed embodiments and other communications terminals, such as another mobile communications terminal 355 or a stationary telephone 380. The mobile communications terminals 350, 355 are connected to a mobile telecommunications network 310 through Radio Frequency links via base stations 340.

The telecommunications system 300 comprises at least one server 330. A server 330 has a data storage and a controller that may be implemented by any commercially available CPU ("Central Processing Unit"), DSP ("Digital Signal Processor") or any other electronic programmable logic device. In one embodiment such a server is a Mobility Management Entity (MME). In one embodiment such a server is a Gateway (GW). The servers 330 are configured to communicate with a mobile telecommunications core network (CN) 310 and/or an external resource 320 such as the internet or a Public Switched Telephone Network (PSTN). A PSTN 320 is configured to communicate with and establish communication between stationary or portable telephones 380. In one embodiment the external resource comprises or is configured to communicate with an external service provider 390. In one embodiment the servers 330 are configured to communicate with other communications terminals using a packet switched technology or protocol. In such an embodiment the servers 330 may make up an Evolved Packet Core (EPC) layer.

The servers 330 are configured to communicate with nodes, also referred to as base stations 340. In one embodiment the base station 340 is an evolved Node Base (eNB). A base station 340 is further configured to communicate with a server 330. In one embodiment the communication between a server 330 and a base station 340 is effected through a standard or protocol 370. In one embodiment the protocol is S1. A base station 340 is configured to communicate with another base station 340. In one embodiment the communication between a base station 340 and another base station 340 is effected through a standard or protocol 360. In one embodiment the protocol 360 is X2. A base station 340 is further configured to handle or service a cell. In one embodiment the at least one base stations 340 make up a Long Term Evolution (LTE) layer. In one embodiment the at least one base stations 340 make up an LTE Advanced layer.

In one embodiment the base station 340 is configured to communicate with a mobile communications terminal 350 (100) through a wireless radio frequency protocol.

In one embodiment the telecommunications system 300 is an Evolved Packet System (EPS) network. In one embodiment the telecommunications system is a system based on the 3GPP (3rd Generation Partnership Project) standard. In one embodiment the telecommunications system is a system based on the UMTS (Universal Mobile Telecommunications System) standard. In one embodiment the telecommunications system is a system based on a telecommunications standard such as GSM, D-AMPS, CDMA2000, FOMA or TD-SCDMA.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other devices. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

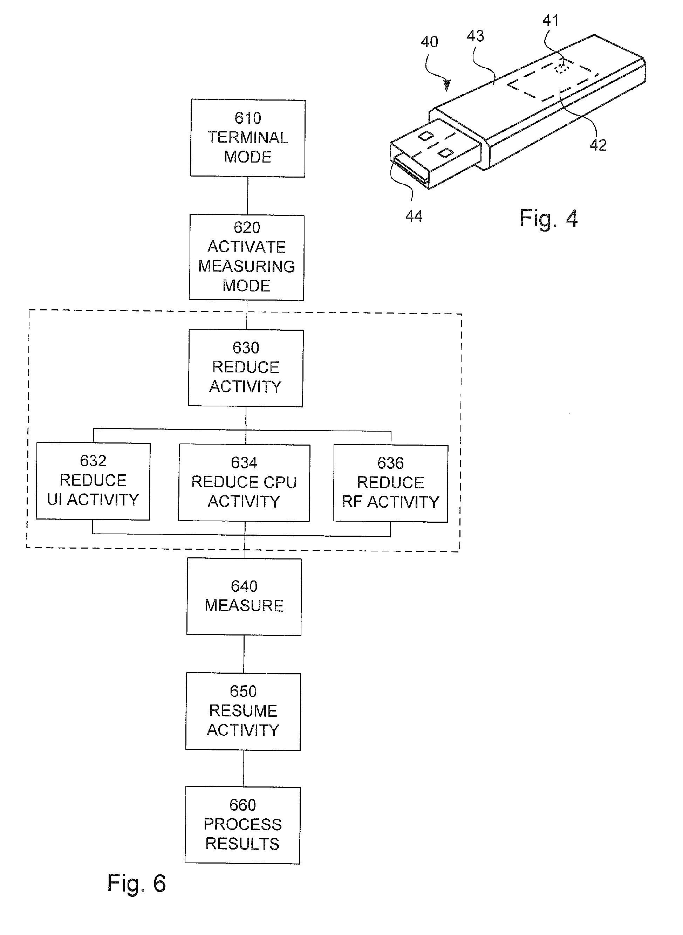

Figure 4 shows a schematic view of a computer-readable medium as described in the above. The computer-readable medium 40 is in this embodiment a memory stick, such as a Universal Serial Bus (USB) stick. The USB stick 40 comprises a housing 43 having an interface, such as a connector 44, and a memory chip 42. The memory chip 42 is a flash memory, that is, a non-volatile data storage that can be electrically erased and re-programmed. The memory chip 42 is programmed with instructions 41 that when loaded (possibly via the interface 44) into a controller, such as a processor, executes a method or procedure according to the embodiments disclosed above. The USB stick is arranged to be connected to and read by a reading device, such as a terminal according to figure 1, for loading the instructions into the controller. It should be noted that a computer-readable medium can also be other mediums such as compact discs, digital video discs, hard drives or other memory technologies commonly used. The instructions can also be downloaded from the computer-readable medium via a wireless interface to be loaded into the controller.

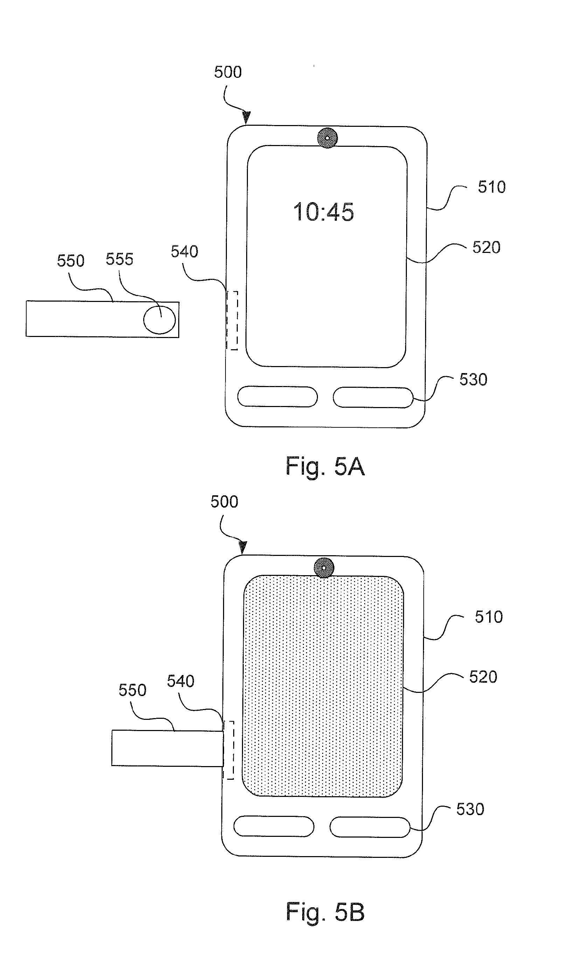

Figure 5A shows a schematic view of an embodiment of the mobile terminal 100, 200 and 350 of figures 1, 2 and 3. The mobile terminal 500 comprises a blood glucose measuring device 540 which is arranged to receive a test strip 550. The test strip 550 carries a test substance 555.

It should be noted that there exists other means of providing the test substance 555 to the blood glucose measuring device 540. Such other means are also taken to be part of the teachings of this description.

The mobile terminal 500, which in this embodiment is a mobile communications terminal 500, is operating in a terminal functional mode. In this example, where the mobile terminal is a mobile communications terminal 500, the terminal functional mode is a telecommunications mode, an idle mode or other mode common to mobile communications terminals. In figure 5A the terminal functional mode is indicated by the display 520 being active and displaying a notification. In this example the notification is simply the time of day. A person skilled in mobile communications terminals would realize that other terminal functional modes also exist and that the mobile communications terminal 500 is arranged to display also other data and information depending on which mode the mobile communications terminal 500 is operating in.

The mobile communications terminal 500 is arranged to enter a measuring mode by user activation of a blood glucose level measuring functionality, for example through selection of a menu option. A person skilled in mobile communications terminals would realize how such a menu option and selection thereof could be implemented. This will thus not be disclosed in detail herein. As the blood glucose level measuring mode is active the mobile communications terminal 500 is arranged to receive the test strip 550 and the test substance 555. The test substance 555 is, in the case of an actual blood glucose level measurement, the blood of a test subject. In the case of a calibration or test, the test substance 555 is a (special) testing substance having known qualities.

The mobile communications terminal 500 is also additionally or alternatively arranged to enter the blood glucose level measuring mode by directly receiving the test strip 550 in the blood glucose measuring device 540. This allows a user to automatically start the measuring by inserting the test strip 550 directly into the blood glucose measuring device 540, without navigating a menu structure, which is beneficial especially for technically novice users.

In one embodiment the mobile communications terminal 500 is arranged so that the menu option can only activate a test procedure for testing the accuracy of the blood glucose measuring device 540 or a calibration process. In such an embodiment the mobile communications terminal 500 is further arranged so that direct insertion of the test strip 550 activates only an actual measurement of a blood sample. This enables a user to more easily ensure that the wrong kind of procedure is not accidentally initiated, thereby avoiding for example an accidental recalibration of the blood glucose measuring device 540.

To reduce the interference caused by external sources to the measurement to be undertaken by the blood glucose measuring device 540, the mobile communications terminal 500 is configured to adapt its operation accordingly during the measuring mode. In one embodiment the mobile communications terminal 500 is configured to reduce the illumination level of the display 520 during the blood glucose level measuring mode. The reduced illumination level of the display 520 causes less radiation to be generated by the display 520 and the interference is thereby reduced. Also, the reduced illumination level for the display 520 requires less power to operate which causes less heat to be generated and thus reduces the interference caused by the surrounding temperature. The reduction in intensity also reduces the battery power consumption of the mobile communications terminal 500.

In one embodiment the mobile communications terminal 500 is configured to reduce the illumination level for the display 520 by deactivating the display 520. By deactivating the display 520 the interference is further reduced as the data communication between the controller (not shown in figure 5A or 5B, but referenced 210 in figure 2) and the display 520 is shut down. This improves the quality of the blood glucose level measurement. In figure 5B, which shows a schematic view of a mobile terminal 500 according to herein (in this example a mobile communications terminal 500) the display 520 is indicated to be deactivated by being shown as blacked out.

In one embodiment the keypad 530 of the mobile communications terminal 500 is also deactivated, partially or fully. This reduces the interference in that no signals are sent from the keys 530 to the controller (210). This is especially beneficial for hard-wired keys as they transmit activation signals through PCB (Printed Circuit Board) and other wiring acting as antennas. In such an embodiment the blood glucose level measuring mode may be cancelled by removing the test strip 550 from the blood glucose measuring device 540 or when the measuring device 540 has performed the measurement.

The mobile communications terminal 500 is thus configured to reduce the interference caused by deactivating or reducing the activity of user interface components 520, 530.

In one embodiment the mobile communications terminal 500 is further configured to deactivate components which consume a lot of power. Such high power components usually cause a lot of electromagnetic disturbance and especially if they transmit or receive data communication (even internally). By deactivating those components the interference is significantly reduced and it further reduces the interference as less heat is generated by the high power component of the mobile communications terminal 500. This also reduces the power consumption of the mobile communications terminal 500. One example of such a component is the radio frequency (RF) interface (not shown in figure 5A or 5B, but referenced 230 in figure 2). Deactivating the RF interface, partially (for example deactivating WiFi or Bluetooth® while maintaining contact with a cellular network operator) or fully, also carries the benefit that the interference is further reduced as the RF interference is significantly reduced. Other examples of high power components are cameras, media players and infrared ports.

In one embodiment the mobile communications terminal 500 is configured to activate a flight mode while in the measuring mode.

Reducing the activity of some components and thereby reducing the heat generated by those components also enables the temperature to be maintained on a steady level and prevents it from fluctuating (between and during measurements), thereby making it easier to ensure that calibrations and measurements are accurate.

In one embodiment the mobile communications terminal 500 is configured to reduce the activity of the controller (210) by deactivating some processes being executed by the controller (210). In one embodiment, where the blood glucose measuring device 540 comprises a dedicated controller (not shown), the controller (210) may be turned off completely or put in a standby mode.

By reducing the activity of the controller the interference caused by the operation of the controller (210) is reduced. The operation of a controller (210) constitutes much data communication with other components such as memory, both internal, cache and external (hard drives), and all such data communication generates additional interference for the blood glucose measuring device 540. The processor clock is also a source of interference and if the controller (210) is turned off, the clock may also be turned off thereby reducing the interference to the blood glucose measuring device 540. This is possible if, in the blood glucose level measuring mode, no other activities are possible or if only a limited set of applications are allowed to execute actively. The battery consumption of the mobile communications terminal 500 is also further reduced by reducing the activity of the controller (210).

In one embodiment the mobile communications terminal 500 is configured to turn off the mobile communications operations and components and only maintain the blood glucose measuring device 540 active. This allows for a minimum of interference to be caused to the measurement.

In one embodiment the measuring device 540 is configured to determine that enough test substance 555 is carried on the test strip 550 to perform an accurate measurement. Should the measuring device 540 determine that the amount of test substance 555 is not adequate it may cause a notification, visual and/or audible, to be effected informing a user that the amount of test substance 555 is insufficient.

As the measurement has been performed the mobile communications terminal 500 resumes its activity and processes the results of the measurement.

The processing may include calculating and displaying the resulting blood glucose level on the display 520, reporting the blood glucose level to a third party through the RF interface, use the blood glucose level in an application, such as an insulin injecting or portioning application, or a combination thereof. It should be noted that the processing of the results may be performed while one or more components are still operating in a reduced manner. For example, the display may be kept dark while the processing is performed to further save on battery power.

The mobile communications terminal 500 may also store the processed results in a history log for later reference. This allows a user or an application to monitor changes and trends in the blood glucose level of the user and determine if any action should be taken.

To enable the cost of the blood glucose measuring device 540 to be as low as possible, the mobile communications terminal 500 may be arranged to perform the processing of the results in the controller (210).

The mobile communications terminal 500 may also be arranged with a shield (not shown) which shields the blood glucose measuring device 540 from interference.

The mobile communications terminal 500 may also be arranged to measure the temperature at the blood glucose measuring device 540 and to take the measured temperature into account when performing the measurement and processing the results of the measurement.

Figure 6 shows a flowchart of a general method according to herein. A mobile terminal operates in a terminal functional mode 610 and the measuring mode is activated 620. In response thereto the mobile terminal reduces 630 the activity of at least one component. One alternative is to reduce 632 the activity of a user interface component, such as by reducing the illumination level of the display (possibly turning it off). Another alternative is to reduce 634 the activity of the controller (possibly turning it off). Yet an alternative is to reduce 636 the activity of the RF interface (possibly turning it off fully or partially). As the activity of some components has been reduced the mobile terminal performs the measurement 640 and then, as the measurement has been secured, the mobile terminal resumes its activities 650 and processes the results 660 of the measurement.

One benefit is that the manner taught herein reduces the interference caused to the measurement thereby making the measurement more reliable and accurate, which is of importance when performing health measurements.

Another benefit is that by reducing the activity the power consumption of the mobile terminal is reduced, making the battery last longer.

Another benefit is that by reducing the activity of some components, which may not always be active or whose operation varies (such as the display and the RF interface) the mobile terminal maintains a more stable (as in more or less always similar) temperature during the measurement.

The invention has mainly been described above with reference to a few embodiments. However, as is readily appreciated by a person skilled in the art, other embodiments than the ones disclosed above are equally possible within the scope of the invention, as defined by the appended patent claims.

## Claims

1. A mobile terminal (100, 200, 350, 355, 500) comprising a memory (240), a measuring device (540) and a plurality of components (120, 220, 520, 530, 230, 210), one component being a controller (210), wherein said controller (210) is configured to:
operate in a terminal functional mode;
determine that a measuring mode is to be activated and enter said measuring mode;
reduce the activity of at least one of said components (120, 220, 520, 530, 230, 210) thereby reducing the interference caused by said components (120, 220, 520, 530, 230, 210) to said measuring device (540);
cause said measuring device (540) to perform a measurement; and
resume the activity of said at least one component (120, 220, 520, 530, 230, 210).

2. The mobile terminal (500) according to claim 1, wherein the controller (210) is further configured to reduce the activity of the at least one component (120, 220, 520, 530, 230, 210) by reducing the activity of a user interface component (120, 130, 520, 530).

3. The mobile terminal (500) according to claim 2, wherein the controller (210) is further configured to reduce the activity of the user interface component (120, 220, 520, 530, 230, 210) by reducing an illumination level of a display (520).

4. The mobile terminal (500) according to any of claims 1 to 3, wherein the controller (210) is further configured to reduce the activity of the at least one component (120, 220, 520, 530, 230, 210) by reducing the activity of the controller (210).

5. The mobile terminal (500) according to any of claims 1 to 4, wherein the controller (210) is further configured to reduce the activity of the at least one component (120, 220, 520, 530, 230, 210) by reducing the activity of a radio frequency interface (230).

6. The mobile terminal (500) according to claim 5, wherein the controller (210) is further configured to reduce the activity of the radio frequency interface (230) by reducing the activity of a short range, local connectivity component, such as a Bluetooth® or IEEE 802.11 interface.

7. The mobile terminal (500) according to any of claims 1 to 6, wherein reducing said activity of said at least one component includes shutting off said at least one component (120, 220, 520, 530, 230, 210).

8. The mobile terminal (500) according to any of claims 1 to 7, wherein reducing said activity of said at least one component includes reducing a communication between said at least one component (120, 220, 520, 530, 230, 210) and at least another of said components (120, 220, 520, 530, 230, 210).

9. The mobile terminal (500) according to any of claims 1 to 8, wherein the controller (210) is further configured process the results of the measurement.

10. The mobile terminal (500) according to any of claims 1 to 9, wherein the mobile terminal (500) is a mobile communications terminal (100, 200, 350, 500).

11. The mobile terminal (500) according to claim 10, wherein the mobile communications terminal (100, 200, 350, 500) is a mobile phone (100, 200, 350, 500).

12. The mobile terminal (500) according to any of claims 1 to 11, wherein the measuring device (540) is a blood glucose measuring device (540).

13. A method for use in a mobile terminal (100, 200, 500) comprising a memory (240), a measuring device (540) and a plurality of components (120, 220, 520, 530, 230, 210), said method comprising:
operating in a terminal functional mode;
determining that a measuring mode is to be activated and enter said measuring mode;
reducing the activity of at least one of said components (120, 220, 520, 530, 230, 210) thereby reducing the interference caused by said components (120, 220, 520, 530, 230, 210) to said measuring device (540);
causing said measuring device (540) to perform a measurement; and
resuming the activity of said at least one component (120, 220, 520, 530, 230, 210).

14. A computer readable storage medium (40) encoded with instructions (41) that, when loaded and executed on a processor, causes the method according to claim 13 to be performed.
